(19) Europäisches Patentamt
European Patent Office
Office européen des brevets

(11) Veröffentlichungsnummer: **0 148 730**
**B1**

(12) **EUROPÄISCHE PATENTSCHRIFT**

(45) Veröffentlichungstag der Patentschrift: **23.08.89**

(51) Int. Cl.⁴: **C 07 D 213/64**, D 06 M 16/00

(21) Anmeldenummer: **84810632.4**

(22) Anmeldetag: **17.12.84**

(54) **Verfahren zum Schützen von Keratinmaterial vor dem Befall durch keratinfressende Insekten und neue Pyridyloxytrifluormethansulfonanilide.**

(30) Priorität: **23.12.83 CH 6885/83**

(43) Veröffentlichungstag der Anmeldung: **17.07.85 Patentblatt 85/29**

(45) Bekanntmachung des Hinweises auf die Patenterteilung: **23.08.89 Patentblatt 89/34**

(84) Benannte Vertragsstaaten: **BE CH DE FR GB IT LI NL**

(56) Entgegenhaltungen:
**DE-B-1 058 049**
**US-A-3 066 166**
**US-A-3 686 192**

(73) Patentinhaber: **CIBA- GEIGY AG, Klybeckstrasse 141, CH- 4002 Basel (CH)**

(72) Erfinder: **Böger, Manfred, Wilhelm Glock- Strasse 14, D-7858 Weil am Rhein 5 (DE)**
Erfinder: **de Sousa, Bernardo, Dr., Paradiesstrasse 15, CH- 4125 Riehen (CH)**
Erfinder: **Reinehr, Dieter, Dr., Wolfsheule 10, D-7842 Kandern (DE)**
Erfinder: **Schmid, Werner, Keltenweg 40, CH- 4125 Riehen (CH)**
Erfinder: **Rempfler, Hermann, Dr., Brücklismattstrasse 16, CH- 4107 Ettingen (CH)**
Erfinder: **Tobler, Hans, Dr., Baselmattweg 157, CH- 4123 Allschwil (CH)**

**EP 0 148 730 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein Verfahren zum Schützen bzw. Ausrüsten von Keratinmaterial vor bzw. gegen Befall durch keratinfressende Insekten durch Behandlung dieser Materialien mit bestimmten Pyridyloxytrifluormethansulfonaniliden; ferner neue Pyridyloxytrifluormethansulfonanilide, Verfahren zu deren Herstellung, diese enthaltende Mittel und deren Verwendung als Motten- und Käferschutzmittel.

Aus der DE-A-890 883, DE-A-1 058 049 und US-A-3 066 166 sind Chlormethansulfonamide mehrkerniger Amine, u.a. halogenierte Phenoxychlormethansulfonanilide bekannt, die als Schutzmittel gegen Textilschädlinge sowie gegen Schimmel- und Bakterienbefall eingesetzt werden können.

Es wurde überraschenderweise gefunden, dass bestimmte Pyridyloxytrifluormethansulfonanilide sich besonders gut als Schutzmittel gegen keratinfressende Insekten eignen. Einige Pyridyloxytrifluormethansulfonanilide sind aus der US-A-3 686 192 bekannt. Sie werden dort als herbizid und antiinflammatorisch wirksame Substanzen beschrieben.

Das erfindungsgemässe Verfahren zum Schützen bzw. Ausrüsten von Keratinmaterial bzw. keratinhaltigem Material vor bzw. gegen Befall durch keratinfressende Insekten ist dadurch gekennzeichnet, dass man das zu schützende Material mit Verbindungen der Formel (1)

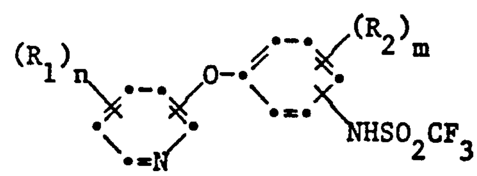

(1)

oder deren Salzen behandelt, worin

$R_1$ und $R_2$ unabhängig voneinander jeweils Halogen, Halogenalkyl, Alkyl, Nitro, Cyano, Alkoxy, Halogenalkoxy oder Alkoxycarbonyl und

n und m unabhängig voneinander 0 oder eine Zahl von 1 bis 3 bedeuten,

wobei, wenn n bzw. m > 1, die Substituenten $R_1$ bzw. $R_2$ gleich oder verschieden sein können und wobei im Molekül mindestens ein Substituent aus der Gruppe Halogen, Halogenalkyl und Halogenalkoxy enthalten ist.

Weiter betrifft die Erfindung die Verwendung von Verbindungen der Formel (1) als Schutzmittel für Keratinmaterial bzw. keratinhaltiges Material gegen keratinfressende Insekten sowie das mit Hilfe von Verbindungen der Formel (1) geschützte bzw. ausgerüstete Material.

In Formel (1) sind unter Halogen alle Halogenatome zu verstehen, insbesondere Chlor, Brom und Fluor. Bevorzugt ist Chlor. Unsubstituierte und substituierte Alkylgruppenund Alkoxygruppen weisen insbesondere 1 bis 6 C-Atome, vorzugsweise 1 bis 4 C-Atome auf. Halogenalkyl- und -alkoxygruppen enthalten vorzugsweise Chlor oder/und Fluor als Halogenatome. Alkoxycarbonylgruppen weisen 2 bis 5 C-Atome vorzugsweise 2 C-Atome auf.

Die Wirkstoffe enthalten im Molekül neben der $-NHSO_2CF_3$-Gruppe mindestens einen Halogen- oder halogenhaltigen Substituenten. In bevorzugten Verbindungen beträgt die Gesamtzahl der Substituenten $R_1$ + $R_2$ 1 bis 4 (n + m = 1 bis 4), vorzugsweise 2 bis 4 (n + m = 2 bis 4). Sind im Pyridin- bzw. Phenylkern mehrere Substituenten vorhanden ($R_1$ oder $R_2$), so können diese selbstverständlich gleich oder verschieden sein. Von den Substituenten Halogenalkyl, Nitro, Cyano und Halogenalkoxy sind in den Verbindungen der Formel (1) vorzugsweise höchstens 3, insbesondere höchstens 2 und besonders bevorzugt höchstens einer vorhanden. Die genannten Substituenten treten dabei vorzugsweise als Substituenten $R_1$ auf.

Die Wirkstoffe der Formel (1) können im erfindungsgemässen Verfahren auch in Form ihrer Salze eingesetzt werden. Unter diesen Salzen sind insbesondere die Alkalimetall- und Ammoniumsalze (inklusive substituierte, sich von Aminen ableitende Ammoniumsalze) zu erwähnen. Bevorzugt sind z. B. Natrium-, Kalium- und Ammoniumsalze.

Im erfindungsgemässen Verfahren werden als Verbindungen der Formel (1) vorteilhaft solche eingesetzt, worin für den Fall, dass $R_1$ oder $R_2$ für Halogen steht, die Summe n + m mindestens 2 beträgt.

Insbesondere werden solche Verbindungen der Formel (1) verwendet, worin $R_1$ und $R_2$ unabhängig voneinander Chlor, Fluor, $C_1$-$C_4$-Halogenalkyl, worin Halogen für Chlor oder/und Fluor steht, $C_1$-$C_4$-Alkyl, $C_1$-$C_4$-Alkoxy, $C_2$-$C_4$-Alkoxycarbonyl, Nitro oder Cyano bedeuten, vor allem solche, worin mindestens einer der Substituenten $R_1$ oder $R_2$, vorzugsweise $R_1$, Chlor oder $C_1$-$C_4$-Halogenalkyl, vorzugsweise Trifluormethyl, bedeutet, wobei vorzugsweise einer der Substituenten $R_1$ für $C_1$-$C_4$-Halogenalkyl, insbesondere für Trifluormethyl, steht.

In bevorzugten Verbindungen der Formel (1) beträgt die Summe n + m 1 bis 4, insbesondere 2 bis 4. Ferner sind jene Verbindungen der Formel (1) hervorzuheben, in denen die $NHSO_2CF_3$-Gruppe in 4-Stellung steht, wobei von solchen Verbindungen jene besonders zu erwähnen sind, in denen eine oder beide ortho-Stellung(en) zur $-NHSO_2CF_3$-Gruppe durch $C_1$-$C_4$-Alkyl, vorzugsweise Methyl, besetzt sind.

Ferner werden im erfindungsgemässen Verfahren besonders vorteilhaft solche Verbindungen der Formel (1) eingesetzt, worin der Pyridinring über die 2-Stellung mit dem Sauerstoffatom verknüpft ist.

Praktisch wichtige Verbindungen der Formel (1), die im erfindungsgemässen Verfahren eingesetzt werden können, entsprechen der Formel (2)

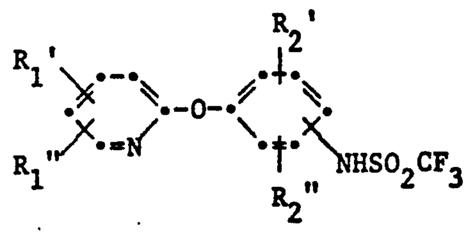

(2) ,

worin

$R_1'$ Chlor oder $C_1$-$C_4$-Halogenalkyl, worin Halogen für Chlor oder/und Fluor steht vorzugsweise Trifluormethyl,

$R_1''$ Wasserstoff, Chlor oder $C_1$-$C_4$-Alkyl,

$R_2'$ Wasserstoff, $C_2$-$C_4$-Alkoxycarbonyl, $C_1$-$C_4$-Alkyl oder Chlor und

$R_2''$ Wasserstoff, $C_1$-$C_4$-Alkyl oder Chlor bedeuten, wobei solche der Formel (3)

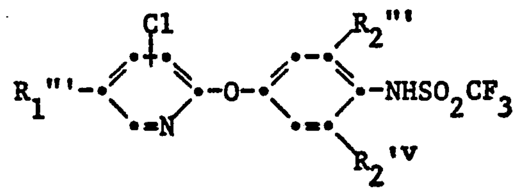

(3) ,

worin

$R_1'''$ Trifluormethyl oder Chlor und $R_2'''$ und $R_2'^{v}$ unabhängig voneinander Wasserstoff, $C_1$-$C_4$-Alkyl oder $-COOCH_3$ bedeuten, besonders gute Ergebnisse liefern.

Die im erfindungsgemässen Verfahren zum Schützen von Keratinmaterial gegen keratinfressende Insekten verwendbaren Verbindungen der Formel (1) wirken insbesondere z. B. gegen keratinfressende Larven von Lepidoptera, z. B. Tineola spec. und Tinea spec. sowie gegen keratinfressende Larven von Coleoptera, z. B. Anthrenus spec. und Attagenus spec. Sie eignen sich vorzüglich zum Schützen von keratinischem bzw. keratinhaltigem Material gegen Insektenfrass, insbesondere zur wasch und lichtechten Ausrüstung gegen Insekten, insbesondere zur Motten und Käferechtausrüstung von derartigen Materialien. Es kann keratinisches bzw. keratinhaltiges Material sowohl in rohem als auch in verarbeitetem Zustand ausgerüstet werden, z. B. rohe oder verarbeitete Schafwolle, Produkte aus anderen Tierhaaren, Felle, Pelze und Federn.

Praktisch besonders wichtig ist die Wirksamkeit der Verbindungen der Formel (1) gegen die Larven der Kleidermotte (Tineola bisselliella), der Pelzmotte (Tinea pellionella) und der Samenmotte (Hofmannophila pseudopretella) sowie gegen die Larven der Pelz- und Teppichkäfer (Attagenus spec. bzw. Anthrenus spec.), z. B. des Wollkraut-Blütenkäfers (Anthrenus verbasci), des Bibernell-Blütenkäfers (Anthrenus pimpinellae), des Gemeinen Teppichkäfers (Anthrenus scrophulariae), des Bebänderten Teppichkäfers (Anthrenus fasciatus), des Gefleckten Pelzkäfers (Attagenus pellio) und vor allem des Dunklen Pelzkäfers (Attagenus piceus) und des Teppichkäfers (Anthrenus flavipes).

Bevorzugt wird das erfindungsgemässe Verfahren einerseits zum Schützen von Textilien aus Wolle, z. B. von Wolldecken, Wollteppichen, Wollwäsche, Wollkleidern und Wirkwaren bzw. wollhaltigen Textilien, wie Mischgeweben, deren eine Komponente Wolle ist, z. B. Mischgeweben aus Wolle und anderen Naturfasern, vorzugsweise Baumwolle oder aus Wolle und Kunstfasern, andererseits auch zum Schützen von Pelzen und Fellen vor dem Befall durch die erwähnten Schädlinge eingesetzt.

Die Verbindungen der Formel (1) werden auf die oben erwähnten Substrate, insbesondere auf wollene und wollhaltige Textilien, vorzugsweise mit Hilfe der aus der Färbereipraxis bekannten Methoden wie Ausziehfärbeverfahren und Foulardapplikation aufgebracht. Zu diesem Zweck wird eine wässrige Lösung oder Dispersion (bzw. Emulsion oder Suspension) des jeweiligen Wirkstoffes angefertigt. Der Wirkstoff kann vorher in organischen Lösungsmitteln wie aliphatischen und alicyclischen Alkoholen, Ketonen, Kohlenwasserstoffen wie Benzol, Xylolen, Toluol, Benzinen, ferner chlorierten und fluorierten Kohlenwasserstoffen, insbesondere in Propylenglykol, Methoxyäthanol, Äthoxyäthanol oder Dimethylformamid gelöst und anschliessend dem Applikationsbad zugegeben werden, das zusätzliche in der Färbereipraxis übliche Hilfsstoffe, z. B. Dispergatoren, Netzmittel, Säuren, Basen und/oder Farbstoffe enthalten kann, wobei derartige Hilfsstoffe bereits in der organischen Stammformulierung enthalten sein können.

Die Textilmaterialien können z. B. durch heisse oder kalte wässrige Färbe-, Bleich-, Chromierungs- oder Nachbehandlungsbäder mit den Wirkstoffen imprägniert werden, wobei verschiedene Textilausrüstungsverfahren, wie z. B. das Foulard- oder Ausziehverfahren, in Frage kommen.

Die Behandlung erfolgt zweckmässig bei Temperaturen von 10 bis 100°C, im Färbebad vorzugsweise bei etwa 60 - 100°C, im Nachbehandlungs- oder Waschbad bei vorzugsweise 10 bis 70, insbesondere 20 bis 60°C.

Als zusätzliche Hilfsmittel können den Applikationsflotten z. B. Dispergatoren, Emulgatoren oder Tenside

zugegeben werden. Daneben kann die Flotte auch noch übliche Hilfsstoffe, wie wasserlösliche Perborate, Polyphosphate, Carbonate, Silikate, optische Aufheller, Weichmacher, sauer reagierende Salze, wie Ammonium- oder Zinksilikonfluorid, oder gewisse organische Säuren, wie Oxalsäure, Essigsäure oder besonders Ameisensäure, ferner Antimikrobika und Appreturmittel, z. B. solche auf Kunstharzbasis oder Stärke, enthalten. Falls die Motten- und Käferechtausrüstung gemeinsam mit dem Färben des Materials (z. B. Wolle) durchgeführt wird, enthalten die Flotten auch noch die entsprechenden Farbstoffe und gegebenenfalls die dazu erforderlichen Hilfsmittel, z. B. Egalisiermittel.

Wässrige Applikationsflotten können z. B. Tenside, beispielsweise anionaktive Verbindungen, wie Seifen und andere Carboxylate (z. B. Alkalisalze höherer Fettsäuren), Abkömmlinge von Schwefel-Sauerstoffsäuren (z. B. Natriumsalz der Dodecylbenzolsulfonsäure, wasserlösliche Salze von Schwefelsäuremonoestern höhermolekularer Alkohole oder ihrer Polyglykoläther, wie etwa lösliche Salze von Dodecylalkoholsulfat oder von Dodecylalkoholpolyglykoläther-sulfat), Abkömmlinge von Phosphor-Sauerstoffsäuren (z. B. Phosphate), Abkömmlinge mit saurem (elektrophilem) Stickstoff in der hydrophilen Gruppe (z. B. Disulfinsalze), kationaktive Tenside, wie Amine und ihre Salze (z. B. Lauryl-diäthylentriamin), Oniumverbindungen, Aminoxide oder nichtionogene Tenside, wie Polyhydroxyverbindungen, Tenside auf Mono- oder Polysaccharidbasis, höhermolekulare Acetylenglykole, Polyglykoläther (z. B. Polyglykoläther höherer Fettalkohole, Polyglykoläther höhermolekular-alkylierter Phenole) enthalten.

Im Falle von nicht-wässriger Applikation (Lösungsmittelapplikation) kann ein entsprechender Teil einer Verbindung der Formel (1) auch einem geeigneten Lösungsmittel zugegeben werden und mit der so erhaltenen Lösung kann das zu schützende Material imprägniert werden. Als Lösungsmittel kommen hierfür unter anderen Trichloräthylen, Methylenchlorid, Kohlenwasserstoffe, Propylenglykol, Methoxyäthanol, Aethoxyäthanol, Dimethylformamid in Frage, denen noch Verteilungsmittel (z. B. Emulgatoren, wie sulfiertes Ricinusöl, Fettalkoholsulfate usw.) und/oder andere Hilfsstoffe zugesetzt werden können. Die zu schützenden Materialien werden üblicherweise mit diesen Lösungen einfach imprägniert.

Die Ausrüstung der zu schützenden Materialien kann aber auch mit einem Trockenreinigungsprozess kombiniert werden. Ein entsprechender Teil einer Verbindung der Formel (1) wird zu diesem Zweck im Reinigungsmittel (etwa niedere halogenierte Alkane, z. B. Trichloräthylen usw.) gelöst und der Reinigungsprozess wird wie üblich durchgeführt.

Ein Anteil einer Verbindung der Formel (1) kann aber auch in leicht flüchtigen organischen Lösungsmitteln gelöst werden und diese Lösung kann dann auf das zu schützende Substrat aufgesprüht werden (Sprühapplikation). Für diese Applikationsart eignen sich vor allem wollhaltige Textilien, Pelze und Federn. Der Vorteil der Sprühapplikation besteht darin, dass wegen der Rückgewinnung der Lösungsmittel eine Belastung der Abwässer vermieden wird.

Im erfindungsgemässen Verfahren können die Verbindungen der Formel (1) auch in Kombination mit anderen gegen keratinfressende Insekten wirksamen Schutzmitteln angewendet werden, z. B. in Kombination mit Harnstoffderivaten, Benzimidazolen, aromatischen Sulfonamiden, Phosphor und Phosphonsäureestern, synthetischen Pyrethroiden und 5-Phenylcarba moylbarbitursäurederivaten.

Die jeweils eingesetzte Menge an Verbindung der Formel (1), die in das jeweilige Applikationsbad bzw. das nichtwässrige Lösungsmittel eingebracht wird, hängt vom jeweiligen Substrat und der Applikationsmethode ab. Diese Menge wird üblicherweise jedoch so bemessen, dass nach dem Aufziehen auf das jeweils zu schützende Material letzteres etwa 10 bis 2000 ppm, vorzugsweise 100 bis 1000 ppm an Verbindung der Formel (1) enthält, wobei die obere Grenze weitgehend durch Überlegungen ökonomischer Natur gegeben ist, während die untere Grenze von Kriterien wie angestrebte Breite und Dauerhaftigkeit der Schutzwirkung abhängt. Dies ergibt z. B. für das Ausziehverfahren bei einem Flottenverhältnis von 1 : 20 Konzentrationen von 0,001 bis 1 g Wirksubstanz/l Behandlungsbad, je nach erreichbarem Ausziehgrad. Beim Foulardverfahren sind Konzentrationen bis 2 g Wirksubstanz pro Liter möglich.

Die Erfindung betrifft insbesondere auch neue Pyridyloxytrifluormethansulfonanilide der Formel (1a)

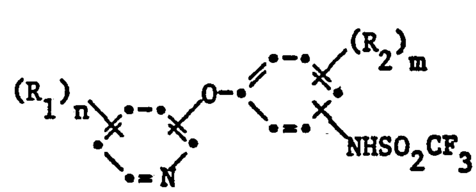 (1a)

und deren Salze, worin

$R_1$ und $R_2$ unabhängig voneinander jeweils Halogen, Halogenalkyl, Alkyl, Nitro, Cyano, Alkoxy, Halogenalkoxy oder Alkoxycarbonyl und

n und m unabhängig voneinander 0 oder eine Zahl von 1 bis 3 bedeuten,

wobei, wenn n bzw. m > 1, die Substituenten $R_1$ bzw. $R_2$ gleich oder verschieden sein können und wobei im Molekül mindestens ein Substituent aus der Gruppe Halogen, Halogenalkyl und Halogenalkoxy enthalten ist,

und wobei für den Fall, dass $R_1$ oder $R_2$ für Halogen steht, die Summe n + m mindestens 2 beträgt.

Für die einzelnen Substituenten und die Salze der neuen Verbindungen gelten die im Anschluss an die Formel (1) gegebenen näheren Erklärungen.

Die Verbindungen der Formel (1a) enthalten im Molekül neben der $NHSO_2CF_3$-Gruppe mindestens einen

Halogen- oder halogenhaltigen Substituenten. In bevorzugten Verbindungen beträgt die Gesamtzahl der Substituenten $R_1 + R_2$ 1 bis 4 (n + m = 1 bis 4), viorzugsweise 2 bis 4 (n + m = 2 bis 4). Sind im Pyridin- bzw. Phenylkern mehrere Substituenten vorhanden ($R_1$ oder $R_2$), so können diese selbstverständlich gleich oder verschieden sein. Von den Substituenten Halogenalkyl, Nitro, Cyano, Halogenalkoxy und Alkoxycarbonyl sind in den Verbindungen der Formel (1a) vorzugsweise höchstens 3, insbesondere höchstens 2 und besonders bevorzugt höchstens einer vorhanden. Die genannten Substituenten treten dabei vorzugsweise als Substituenten $R_1$ auf.

Besonders zu erwähnen sind jene Verbindungen der Formel (1a), in denen $R_1$ und $R_2$ unabhängig voneinander Chlor, Fluor, $C_1$-$C_4$-Halogen-alkyl worin Halogen für Chlor oder/und Fluor steht, $C_1$-$C_4$-Alkyl, $C_1$-$C_4$-Alkoxy, Nitro oder Cyano bedeuten vor allem solche, worin mindestens einer der Substituenten $R_1$ oder $R_2$, vorzugsweise $R_1$, Chlor oder $C_1$-$C_4$-Halogenalkyl, vorzugsweise Trifluormethyl, bedeutet, wobei vorzugsweise einer der Substituenten $R_1$ für $C_1$-$C_4$-Halogenalkyl, vorzugsweise für Trifluormethyl, steht.

In bevorzugten Verbindungen der Formel (1a) beträgt die Summe n + m 1 bis 4, insbesondere 2 bis 4. Ferner sind jene Verbindungen der Formel (1a) hervorzuheben, in denen die $NHSO_2CF_3$-Gruppe in 4-Stellung steht, wobei von solchen Verbindungen jene besonders zu erwähnen sind, in denen eine oder beide ortho-Stellung(en) zur $-NHSO_2CF_3$-Gruppe durch $C_1$-$C_4$-Alkyl, vorzugsweise Methyl, besetzt sind.

Als besonders vorteilhaft haben sich jene Verbindungen der Formel (1a) erwiesen, in denen der Pyridinring über die 2-Stellung mit dem Sauerstoffatom verknüpft ist.

Praktisch besonders wichtig sind Pyridyloxytrifluormethansulfonanilide der Formel (2a)

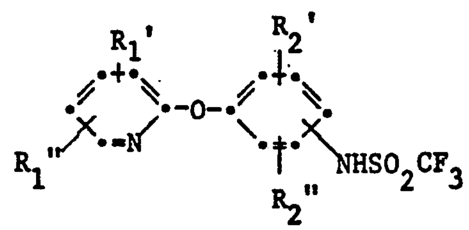

(2a) ,

worin

$R_1'$ Chlor oder $C_1$-$C_4$-Halogenalkyl, worin Halogen für Chlor oder Fluor steht,
$R_1''$ Wasserstoff, Chlor oder $C_1$-$C_4$-Alkyl,
$R_2'$ Wasserstoff, $C_2$-$C_4$-Alkoxycarbonyl, $C_1$-$C_4$-Alkyl oder Chlor und
$R_2''$ Wasserstoff, $C_1$-$C_4$-Alkyl oder Chlor bedeuten, wobei die unter Formel (1a) festgelegten Massgaben bezüglich der Substituentenbedeutungen gelten;

Verbindungen der Formel (3a)

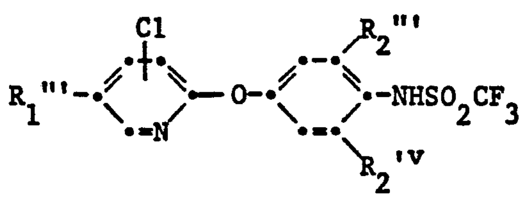

(3a) ,

worin

$R_1'$ Triflourmethyl oder Chlor und
$R_2'''$ und $R_2'^{v}$ unabhängig voneinander Wasserstoff oder $C_1$-$C_4$-Alkyl bedeuten,

zeigen besonders gute Wirkung gegen Keratinschädlinge.

Die im erfindungsgemässen Verfahren einsetzbaren Verbindungen der Formeln (1) - (3) und damit auch die neuen erfindungsgemässen Verbindungen der Formeln (1a) - (3a) können nach an sich bekannten Herstellungsverfahren erhalten werden.

Ein solches Verfahren besteht darin, dass man ein Pyridyloxyanilin der Formel (4)

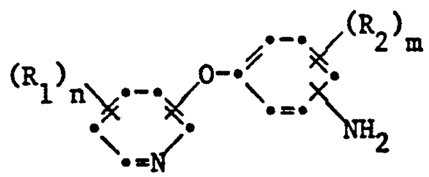

(4) ,

worin die allgemeinen Symbole die in Formel (1) angegebene Bedeutung haben, in Gegenwart einer Base mit Trifluormethansulfonsäureanhydrid oder einem Trifluormethansulfonsäurehalogenid umsetzt.

Von den Trifluormethansulfonsäurehalogeniden sind insbesondere Trifluormethansulfonsäurechlorid und -fluorid geeignet. Bevorzugt ist jedoch die Verwendung von Trifluormethansulfonsäureanhydrid.

Die Umsetzung einer Verbindung der Formel (4) mit Trifluormethansulfonsäurehalogenid oder -anhydrid wird zweckmässigerweise in Gegenwart einer Stickstoffbase, beispielsweise einem tertiären Amin, wie Pyridin, Triäthylamin oder N,N-Dimethylanilin durchgeführt.

Die Umsetzung kann in Anwesenheit von reaktionsinerten Lösungs oder Verdünnungsmitteln durchgeführt werden. In Frage kommen beispielsweise aliphatische und aromatische Kohlenwasserstoffe, wie Cyclohexan, Petroläther, Benzol, Toluol und Xylole; halogenierte Kohlenwasserstoffe, wie Methylenchlorid, Äthylenchlorid, Dichlormethan, Trichlormethan, Chloroform, Tetrachlorkohlenstoff, Tetrachloräthylen und Chlorbenzol; Äther und ätherartige Verbindungen, wie Anisol, Dioxan, Tetrahydrofuran, 1,2-Dimethoxyäthan und Dialkyläther wie Diäthyläther, Diisopropyläther und tert.-Butylmethyläther; Nitrile, wie Acetonitril und Propionitril; N,N-dialkylierte Amide, wie Dimethylformamid und Dimethylacetamid; Dimethylsulfoxid; Ketone, wie Aceton, Diäthylketon und Methyläthylketon; und Gemische solcher Lösungsmittel untereinander.

Die Reaktionstemperaturen liegen zweckmässigerweise zwischen -15° und +150°C, bevorzugt zwischen -5° und 120°C.

Nach einem weiteren Verfahren können die Verbindungen der Formel (1) hergestellt werden, indem man ein Phenolat der Formel (5)

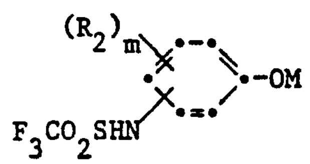

(5) ,

worin

M ein Alkalimetall- oder Kupferatom bedeutet und

$R_2$ und m wie in Formel (1) definiert sind, in einem Lösungsmittel mit einer Verbindung der Formel (6)

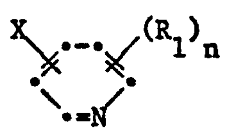

(6)

umsetzt, worin

X für ein Halogenatom steht und

$R_1$ und n wie in Formel (1) definiert sind.

Beispiele für bevorzugte Lösungsmittel in diesem Verfahren sind Pyridin, Chinolin, Dimethylformamid und dergleichen. Bevorzugt wird ein Katalysator verwendet, insbesondere CuCl. Die Alkalimetallsalze (M = Alkalimetallatom) können als solche eingesetzt oder können in situ gebildet werden, z. B. durch Zugabe eines Alkalimetallhydroxids oder -alkoholates. Die Reaktionstemperatur kann je nach der Reaktionsfähigkeit der Ausgangsstoffe in weiten Grenzen variieren, ebenso wie die Reaktionszeiten. Im allgemeinen arbeitet man bei Temperaturen im Bereich von 0 bis 200°C.

Die in den beiden vorstehend beschriebenen Verfahren einzusetzenden Ausgangsverbindungen der Formeln (4) bis (6) sowie die Trifluormethansulfonhalogenide und -anhydride sind bekannt oder können nach an sich bekannten Verfahren erhalten werden. So kann beispielsweise zur Herstellung von Verbindungen der Formel (4) so vorgegangen werden, dass man eine Verbindung der Formel (7)

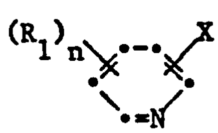

(7) ,

worin

$R_1$ und n wie in Formel (1) definiert sind und

X für ein Halogenatom, vorzugsweise für Fluor und insbesondere für Chlor steht, mit einer Verbindung der Formel (8)

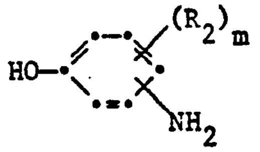 (8)

umsetzt, worin $R_2$ und m wie in Formel (1) definiert sind.

Die Umsetzung von Verbindungen der Formel (7) mit Verbindungen der Formel (8) wird zweckmässigerweise in Gegenwart einer anorganischen oder organischen Base durchgeführt. Beispiele für anorganische Basen sind die Oxide, Hydride, Carbonate und Hydrogencarbonate von Alkali- und Erdalkalimetallen, wie Natriumhydroxyd und Natriumhydrid, für organische Basen vor allem tertiäre Amine wie Triäthylamin, Pyridin und Triäthylendiamin.

Die Umsetzung kann in Gegenwart eines reaktionsinerten Lösungs oder Verdünnungsmittels durchgeführt werden, beispielsweise in Gegenwart eines der vorstehend für die erste Herstellungsmethode von Verbindungen der Formel (1) angeführten Medien. Die Reaktionstemperaturen liegen zweckmässigerweise zwischen 0° und 200° C, bevorzugt zwischen 0° und 150° C.

Die Verbindungen der Formel (5) können leicht durch Umsetzung eines Aminophenols der Formel (8) mit einem Trifluormethansulfonsäurehalogenid oder -anhydrid nach dem ersten erfindungsgemässen Verfahren (Umsetzung der Verbindung der Formel (4) mit den genannten Halogeniden oder Anhydrid) und Überführung des Phenols in ein Salz erhalten werden.

Die Ausgangsprodukte der Formeln (7) und (8) sind bekannt oder lassen sich analog bekannten Methoden herstellen.

Verbindungen der Formel (4) können alternativ auch durch Reduktion der entsprechenden Nitroverbindungen hergestellt werden. Es können dazu übliche Reduktionsmethoden verwendet werden, wie z. B. Eisen (Béchamp-Reduktion), Natriumsulfid und insbesondere katalytische Reduktion mit $H_2$ (Katalysator z. B. Raney-Nickel). Die genannten Nitroverbindungen sind bekannt oder können ebenfalls nach bekannten Verfahren hergestellt werden, z. B. nach dem Schema

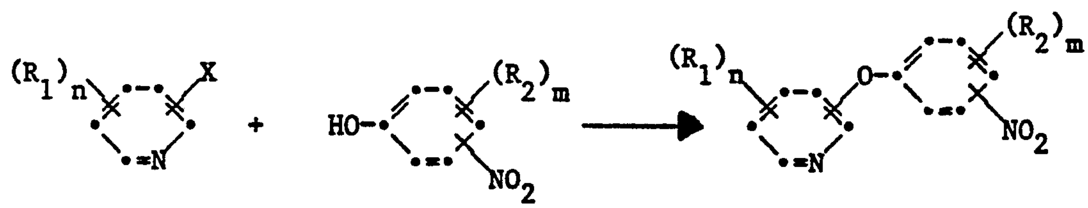

(9) (10) (11)

worin X Chlor, Brom oder Jod bedeutet und die übrigen allgemeinen Symbole wie in Anspruch (1) definiert sind.

Obwohl die Umsetzung in Gegenwart einer Base durchgeführt werden kann, die als Beschleuniger und Säureakzeptor wirkt, wird sie vorzugsweise durch eine Vorreaktion der Verbindung der Formel (10) mit einer Base unter Bildung eines Salzes durchgeführt, wobei die Salze von anorganischen Basen bevorzugt werden. Es ist bekannt, dass solche Salze leicht hergestellt werden, und zwar können sie in situ hergestellt oder isoliert werden. Am meisten werden die Salze von Alkalimetallen, wie z. B. von Natrium und Kalium, oder die Kupfersalze bevorzugt. Wenn Alkalimetallsalze verwendet werden, sind Dimethylformamid und Pyridin die bevorzugten Lösungsmittel. Wenn $R_2$ ein elektronenspendender Substituent, wie z. B. Alkyl oder Alkoxy, in der 2- oder 4-Stellung zur Nitrogruppe ist, ist Pyridin das bevorzugte Lösungsmittel und es wird zweckmässig eine Spur Kupfer-I-chlorid als Katalysator verwendet. Kupfer-I-salze oder ein Kupfer-I-chloridkatalysator und Pyridih als Lösungsmittel werden zur Herstellung von 3-Phenoxynitrobenzolen bevorzugt. Es ist vorteilhaft, wenn X Brom oder Jod ist.

Die vorstehend beschriebenen Zwischenprodukte sind, sofern sie neu sind, ebenso Gegenstand der vorliegenden Erfindung wie die Verfahren zu deren Herstellung.

Die Verbindungen der Formel (1) bzw. (1a) sind sauer (saures H-Atom an der substituierten Aminogruppe). Wie bereits erwähnt, bilden sie daher Salze, die ebenfalls Gegenstand der Erfindung sind bzw. die ebenfalls im erfindungsgemässen Verfahren eingesetzt werden können. Diese Salze sind im allgemeinen Metall-, Ammonium- oder organische Aminsalze (substituierte Ammoniumsalze) und können durch Behandeln der sauren Form mit einer stöchiometrisch äquivalenten Menge einer geeigneten Base unter milden Bedingungen hergestellt werden. Zu den Metallsalzen gemäss der Erfindung gehören Alkalimetall- (z. B. Lithium-, Natrium- und Kalium-), Erdalkalimetall- (z. B. Barium Calcium- und Magnesium-) und Schwermetallsalze (z. B. Zink- und

Eisensalze) sowie auch andere Metallsalze, wie z. B. Aluminiumsalze. Zu geeigneten Basen, die zur Herstellung der Metallsalze verwendet werden können, gehören Metalloxide, -hydroxide, -carbonate, -bicarbonate und -alkoxide. Einige Salze werden auch durch Kationenaustauschreaktionen (durch Umsetzung eines Salzes gemäss der Erfindung mit einem organischen oder anorganischen Salz nach einer Kationenaustauschreaktion) hergestellt. Zu den organischen Aminsalzen gehören die Salze von aliphatischen (z. B. Alkyl-), aromatischen und heterocyclischen Aminen, sowie auch solche mit einer Mischung dieser Strukturarten. Die zur Herstellung der Salze gemäss der Erfindung geeigneten Amine können primär, sekundär oder tertiär sein und enthalten vorzugsweise nicht mehr als 20 Kohlensteffatome. Zu solchen Aminen gehören z. B. Morpholin, Methylcyclohexylamin und dergleichen. Diese Amine und die Ammoniumsalze können durch Umsetzung der sauren Form mit der geeigneten organischen Base oder mit Ammoniumhydroxid hergestellt werden. Geeignete Salze sind im allgemeinen die Alkalimetall-, Ammonium- und Aminsalze.

Die Salze gemäss der Erfindung werden auch häufig durch Umsetzung der Ausgangsverbindungen in wässrigen Lösungen hergestellt. Diese Lösung kann eingedampft werden, um so das Salz der Verbindung im allgemeinen in Form eines trockenen Pulvers, zu ergeben.

In einigen Fällen kann es einfacher sein ein nicht-wässriges Lösungsmittel wie z. B. Alkohole, Aceton usw., zu verdwenden. Die erhaltene Lösung wird dann zur Entfernung des Lösungsmittels entsprechend behandelt, z. B. unter vermindertem Druck eingedampft. Weil viele der Salze wasserlöslich sind, werden sie häufig in der Form der wässrigen Lösungen verwendet.

Verbindungen der Formel (1) bzw. (1a) (z. B. erhalten nach einem der vorstehend beschriebenen Verfahren) können durch geeignete an sich bekannte Reaktionen auch in andere Verbindungen der Formel (1) bzw. (1a) umgewandelt werden.

So können z. B. Verbindungen der Formel (1) bzw. (1a) zur Einführung von Substituenten $R_1$ bzw. $R_2$ halogeniert (vorzugsweise chloriert) oder nitriert werden. Diese Verfahren können nach bekannten Nitrier- und Halogenierungs(Chlorierungs-)verfahren von Aromaten durchgeführt werden. Die Herstellung bzw. Verwandlung von Salzen ist bereits erörtert worden.

Die nachfolgenden Beispiele erläutern das erfindungsgemässe Verfahren sowie die Herstellung der darin verwendeten Wirkstoffe der Formel (1) bzw. der neuen Verbindungen der Formel (1a) näher. Es wird jedoch betont, dass die Erfindung nicht auf diese Beispiele beschränkt ist.

In den nachfolgenden Beispielen, ebenso wie in der gesamten übrigen Beschreibung und in den Patentansprüchen, bedeuten Teileund Prozentangaben Gewichtsteile und Gewichtsprozent, sofern nichts anderes angegeben ist. Schmelzpunkte sind unkorrigiert.

Beispiel 1: 13 g 4-(5-Trifluormethylpyridin-2-yl-oxy)-anilin und 6,1 g N,N-Dimethylanilin in 100 ml Dichlormethan werden bei -15°C mit 14,8 g Trifluormethansulfonsäureanhydrid tropfenweise versetzt. Nachdem sich das Reaktionsgemisch auf Raumtemperatur erwärmt hat, wird es bis zur Auskristallisation stehengelassen. Anschliessend wird das auskristallisierte Produkt abfiltriert und in Essigester aufgenommen. Die Lösung wird zweimal mit 1 N Salzsäure und dann zweimal mit wässriger Natriumchloridlösung gewaschen, getrocknet, mit Aktivkohle entfärbt und eingedampft. Man erhält so 4-(5-Tri-fluormethylpyridin-2-yl-oxy)-trifluormethansulfonanilid der Formel

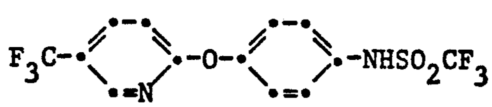 (101)

mit einem Fp. von 162 - 165°C.

Das als Ausgangsprodukt benötigte 4-(5-Trifluormethylpyridin-2-yl-oxy)-anilin kann folgendermassen hergestellt werden:

Zu 22 g (0,2 Mol) 4-Aminophenol in 100 ml Dimethylsulfoxid werden unter Stickstoffatmosphäre 11,2 g (0,2 Mol) Kaliumhydroxid in 15 ml Wasser zugetropft. Bei 25°C werden 36 g (0,2 Mol) 2-Chlor-5-(trifluormethyl)-pyridin in 30 ml Dimethylsulfoxid zugetropft. Das Reaktionsgemisch wird 3 Stunden bei Raumtemperatur gerührt, anschliessend auf Eiswasser gegossen und mit Essigsäureäthylester extrahiert. Die organische Phase wird mit Natriumsulfat getrocknet und eingedampft. Der Rückstand wird in Äther gelöst und mit 1 N Salzsäure extrahiert. Die saure Phase wird mit konzentrierter Natronlauge alkalisch gestellt und mit Äther extrahiert. Die Ätherphase wird mit Natriumsulfat getrocknet und eingedampft. Man erhält so 4-(5-Trifluormethylpyridin-2-yl-oxy)-anilin als braunes Öl.

Beispiel 2: 7,6 g 3-(5-Trifluormethylpyridin-2-yl-oxy)-anilin und 3,6 g N,N-Dimethylanilin in 50 ml Dichlormethan werden bei -15°C mit 8,5 g Trifluormethansulfonsäureanhydrid tropfenweise versetzt. Nachdem sich das Reaktionsgemisch auf Raumtemperatur erwärmt hat, wird das Gemisch mit Essigester verdünnt, zweimal mit 1 N Salzsäure und anschliessend zweimal mit 2 N Natronlauge gewaschen. Die organische Phase wird getrocknet, geklärt und eingedampft. Der kristalline Rückstand wird mit Äther gewaschen, in Essigester gelöst und mit 1 N Salzsäure geschüttelt, dann getrocknet und eingedampft. Man erhält so 3-(5-Trifluormethylpyridin-2-yl-oxy)-trifluormethansulfonanilid der Formel

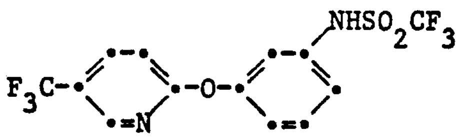

(201)

mit einem Fp. vom 151 - 153°C.

Beispiel 3: Verfährt man analog den in den Beispielen 1 und 2 angegebenen Herstellungsvorschriften, so erhält man unter Verwendung der entsprechenden Ausgangsprodukte die in der nachfolgenden Tabelle zusammengefassten Pyridyloxytrifluormethansulfonanilide der Formel

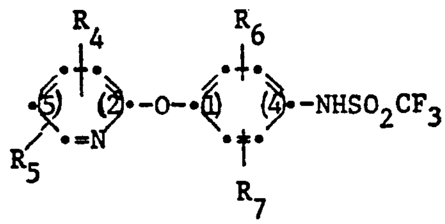

(A)

**Tabelle**

| Verbindung Nr. | $R_4$ | $R_5$ | $R_6$ | $R_7$ | Fp (°C) |
|---|---|---|---|---|---|
| 301 | 5-$CF_3$ | H | 2-$CH_3$ | H | 143 - 145 |
| 302 | 3-C1 | 5-C1 | 2-$CH_3$ | H | 162 - 165 |
| 303 | 5-$CF_3$ | H | 3-$CH_3$ | H | 136 - 137 |
| 304 | 5-$CF_3$ | 3-C1 | H | H | 103 - 105 |
| 305 | 3-C1 | 5-C1 | H | H | 133 - 135 |
| 306 | 5-$CF_3$ | 3-C1 | 3-$CH(CH_3)_2$ | H | 175 - 178 |
| 307 | 5-$CF_3$ | H | 3-$CH(CH_3)_2$ | H | 145 - 147 |
| 308 | 5-$CF_3$ | 3-C1 | 3-$CH_3$ | 5-$CH_3$ | 160 - 162 |
| 309 | 5-$CF_3$ | H | 3-$CH_3$ | 5-$CH_3$ | 154 - 156 |
| 310 | 5-$CF_2CFCl_2$ | 3-Cl | H | H | 134 - 136 |
| 311 | 5-$CF_3$ | H | 2-C1 | 6-C1 | 165 - 168 |
| 312 | 5-$CF_3$ | 3-C1 | 2-C1 | 6-C1 | 127 - 129 |
| 313 | 3-C1 | 5-C1 | 3-$CH_3$ | 5-$CH_3$ | 178 - 180 |
| 314 | 3-C1 | 5-C1 | 3-$CH(CH_2)_2$ | H | 139 - 141 |
| 315 | 3-C1 | 5-C1 | 3-$CH_3$ | H | 148 - 150 |
| 316 | 5-$CF_3$ | 3-C1 | 3-$COOCH_3$ | H | 151 |

In analoger Weise werden auch die Verbindungen der Formel

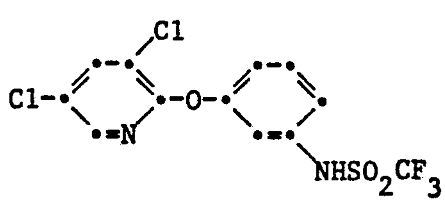

(317)

mit einem Fp. von 172 - 176°C und der Formel

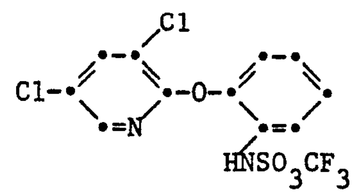

(318)

mit einem Fp. von 90 - 91°C erhalten.

Beispiel 4: Von der Verbindung der Formel (303) wird eine 0,4 %-ige Stammlösung in Äthylenglykolmonomethyläther bereitet. Dann wird bei Zimmertemperatur eine wässrige Applikationsflotte hergestellt, die in 120 ml destilliertem Wasser 0,12 ml eines Netz- und Dispergiermittels, 0,6 ml Ameisensäure 1 : 10 und 0,75 ml der 0,4 %-igen Stammlösung enthält. Dann werden 3 g Wollflanell-Gewebe mit heissem Wasser durchnetzt und bei Zimmertemperatur eingegeben. Unter ständigem Umziehen des Wollmusters wird die Badtemperatur innerhalb 20 Minuten auf 98°C erhöht und 60 Minuten bei 98°C gehalten. Dann wird abgekühlt, die Wollmuster werden zweimal 3 Minuten mit destilliertem Wasser gespült, von Hand abgequetscht und an der Luft getrocknet. Die Wirkstoffkonzentration beträgt 1000 ppm, berechnet auf das Wollgewicht.

Das so getrocknete Muster wird der Mottenechtheitsprüfung (Frassschutz gegen die Kleidermotte Tineola bisselliella Hum.) sowie der Echtheitsprüfung gegen Larven des Pelzkäfers (Attagenus piceus Oliv) und Teppichkäfers (Anthrenus flavipes) gemäss SNV 195 901 unterworfen.

Es werden jeweils 4 Wochen alte Larven von Tineola bisselliella resp. Anthrenus flavipes und 6 bis 7 Wochen alte Larven von Attagenus piceus zur Prüfung verwendet. Aus dem behandelten Wollflanellmuster werden Stücke gleicher Grösse ausgeschnitten und 14 Tage lang bei konstanter Temperatur (28°C) und konstanter relativer Luftfeuchtigkeit (65 %) dem Angriff (Frass) von je 15 Larven des entsprechenden Schädlings ausgesetzt. Die Beurteilung erfolgt einerseits nach dem relativen Gewichtsverlust des Prüflings und andererseits nach der Anzahl noch lebender Organismen.

Die geprüfte Verbindung zeigt eine ausgezeichnete Wirkung gegen alle 3 verwendeten Schädlinge.

Verwendet man an Stelle der Verbindung der Formel (303) eine der Verbindungen der Formeln (101), (201), (301), (304), (306) - (309) und (313) - (315) und verfährt ansonsten wie vorstehend beschrieben, so kommt man ebenfalls zu Wollmustern, die ausgezeichnet gegen die 3 geprüften Schädlinge geschützt sind.

Beispiel 5: Von der Verbindung der Formel (303) wird eine 0,4 %-ige Stammlösung in Äthylenglykolmonomethyläther bereitet. 12,5 ml der Stammlösung werden mit Äthylenglykolmonomethylätherr welcher 0,65 g/l eines Netz- und Dispergiermittels enthält, auf 50 ml verdünnt (=Lösung Nr. 1). 25 ml der Lösung Nr. 1 werden mit Äthylenglykolmonomethyläther, welcher 0,65 g/l eines Netz- und Dispergiermittels enthält, auf 50 ml verdünnt (=Lösung Nr. 2). 25 ml der Lösung Nr. 2 werden erneut mit Äthylenglykolmonomethyläther, welcher 0,5 g/l eines Netz- und Dispergiermittels enthält, auf 50 ml verdünnt (= Lösung Nr. 3).

Von den Lösungen Nr.1, 2 und 3 werden je 3 ml in Kristallisierschalen geleert und je eine geköderte Rondelle aus Wollflanell 3 Sekunden darin benetzt. Die feuchten Rondellen werden anschliessend zwischen Aluminiumfolien foulardiert, und zwar derart, dass die abgequetschten Rondellen je 50 % Flotte aufgenommen haben. Die Konzentration an Wirkstoff auf den Rondellen beträgt dann der Reihe nach 500 ppm, 250 ppm und 125 ppm.

Die feuchten Rondellen werden an der Luft getrocknet und den gleichen biologischen Prüfungen unterworfen, wie in Beispiel 4 beschrieben.

Die geprüfte Verbindung zeigt eine ausgezeichnete Wirkung gegen alle 3 Schädlinge.

Verwendet man an Stelle der Verbindung der Formel (303) eine der Verbindungen der Formeln (101), (201), (301), (304), (306) - (309) und (313) - (316) und verfährt ansonsten wie vorstehend beschrieben, so kommt man ebenfalls zu Wollmustern, die ausgezeichnet gegen die 3 geprüften Schädlinge geschützt sind.

Beispiel 6: Es wird eine 10 %-ige Lösung der Verbindung der Formel (315) in Äthylenglykolmonomethyläther hergestellt. Ein Volumenteil dieser Lösung wird mit 200 Volumenteilen eines zur Trockenreinigung geeigneten Lösungsmittels, z. B. einer passenden Benzinfraktion oder Perchloräthylen, verdünnt. Gewünschtenfalls können noch reinigungsfördernde Zusätze beigefügt werden. Wollartikel werden nun wie üblich in dieser Reinigungsflüssigkeit behandelt und anschliessend auf einen Lösungsmittelgehalt von ca. 100 % des Wollgewichtes abgeschleudert. Sie sind nach dem Trocknen hervorragend gegen die oben genannten keratinfressenden Schädlinge geschützt.

Beispiel 7: Es wird eine 0,5 %-ige Lösung der Verbindung der Formel (315) in Methylenchlorid, Trichloräthylen oder einer tiefsiedenden Benzinfraktion angesetzt. Ein Wollartikel wird mit Hilfe einer üblichen Sprüheinrichtung mit dieser Lösung besprüht, so dass 2 x 15 g/m² an Wirkstofflösung appliziert werden. Bei einem Ausnützungseffekt des Aerosols von 30 % befinden sich dann etwa 400 ppm der Verbindung der Formel (315) auf den Material. Das so ausgerüstete Wollgewebe ist gegen die oben genannten keratinfressenden Schädlinge geschützt.

Verwendet man in den Beispielen 6 und 7 an Stelle der Verbindung der Formel (315) eine der Verbindungen der Formeln (201), (301), (303), (304), (306) - (309), (313) und (314) und verfährt ansonsten wie in den beiden Beispielen beschrieben, so erhält man ebenfalls Wollgewebe, das gegen die oben genannten Keratinschädlinge. geschützt ist.

Beispiel 8: Färbung und gleichzeitige Motten- und Käferechtausrüstung: Auf einem Färbeapparat wird ein Stück Wollgewebe in 600 g einer Färbeflotte, die aus

0,15 g der Verbindung der Formel (303),
30,3 g Glaubersalz,
24,0 g Schwefelsäure conc.,
3,0 g eines roten Farbstoffes der Formel

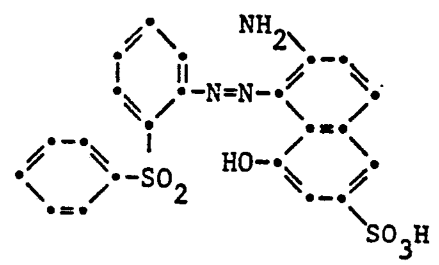

und

541,5 g entmineralisiertem Wasser

besteht, während 5 Minuten bei 40°C vorgenetzt (Flottenverhältnis 1 : 20). Die Flotte wird anschliessend innerhalb 45 Minuten auf ca. 98°C erhitzt. Nach einer einstündigen Behandlungsdauer bei dieser Temperatur wird das Wollgewebe gespült und getrocknet. Der Farbstoff und die Verbindung der Formel (303) sind auf das Gewebe aufgezogen. Das rotgefärbte Wollgewebe ist nach dieser einbadigen Behandlung vollständig gegen Motten- und Käferlarvenfrass geschützt. Dies wird durch Echtheitsprüfung gemäss SNV-Norm 195 901 und 195 902 festgestellt.

Beispiel 9: Anwendung im Nachbehandlungsbad: Auf einem Färbeapparat wird ein Stück Wollgewebe in 400 g einer Nachbehandlungsflotte, die aus

0,1 g der Verbindung der Formel (303),
4 g Ameisensäure 85 % und
395 g entmineralisiertem Wasser

besteht, während 5 Minuten bei 30°C vorgenetzt (Flottenverhältnis 1 : 20). Die Flotte wird dann innerhalb von 20 Minuten auf 45°C erwärmt. Nach einer 30 Minuten dauernden Behandlung bei dieser Temperatur, wobei das Wollgewebe unter ständiger Bewegung gehalten wird, wird letzteres in kaltem Wasser gut gespült und getrocknet. Das so behandelte Wollgewebe ist gegen die Larven von Wollschädlingen vollständig geschützt.

Ersetzt man in den beiden vorstehenden Beispielen 8 und 9 die Verbindung der Formel (303) jeweils durch eine der Verbindungen der Formeln (101), (201), (301), (304), (306) - (309) und (313) - (316), so erhält man ebenfalls gut gegen Wollschädlinge geschütztes Gewebe.

**Patentansprüche**

1. Verfahren zum Schützen bzw. Ausrüsten von Keratinmaterial bzw. keratinhaltigem Material vor bzw. gegen Befall durch keratinfressende Insekten, dadurch gekennzeichnet, dass man das zu schützende Material mit Verbindungen der Formel

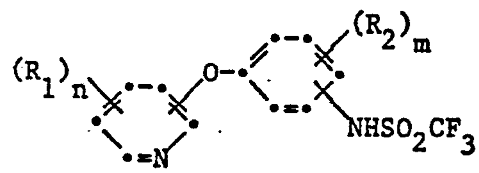

(1)

oder deren Salzen behandelt, worin

$R_1$ und $R_2$ unabhängig voneinander jeweils Halogen worin Halogen Fluor, Chlor, Brom und Jod bedeutet, $C_1$-$C_6$-Halogenalkyl, $C_1$-$C_6$-Alkyl, Nitro, Cyano, $C_1$-$C_6$-Alkoxy, $C_1$-$C_6$-Halogenalkoxy oder $C_2$-$C_5$-Alkoxycarbonyl und

n und m unabhängig voneinander 0 oder eine Zahl von 1 bis 3 bedeuten,

wobei, wenn n bzw. m > 1, die Substituenten $R_1$ bzw. $R_2$ gleich oder verschieden sein können und wobei im Molekül mindestens ein Substituent aus der Gruppe Halogen, Halogen-$C_1$-$C_6$-alkyl und Halogen-$C_1$-$C_6$-alkoxy enthalten ist.

2. Verfahren nach Anspruch 1, dadurch gekennzeichnet, dass man eine Verbindung der Formel (1) einsetzt, worin $R_1$ und $R_2$ unabhängig voneinander Chlor, Fluor, $C_1$-$C_4$-Halogenalkyl, worin Halogen, Chlor oder/und Fluor bedeutet, $C_1$-$C_4$-Alkyl, $C_1$-$C_4$-Alkoxy, $C_2$-$C_4$-Alkoxycarbonyl, Nitro oder Cyano bedeuten.

3. Verfahren nach Anspruch 1, dadurch gekennzeichnet, dass man eine Verbindung der Formel (1) einsetzt, worin für den Fall, dass $R_1$ oder $R_2$ für Halogen steht, die Summe n + m mindestens 2 beträgt.

4. Verfahren nach einem der Ansprüche 1 bis 3, dadurch gekennzeichnet, dass man eine Verbindung der Formel (1) einsetzt, worin die Summe n + m 1 bis 4 beträgt.

5. Verfahren nach einem der Ansprüche 1 bis 4, dadurch gekennzeichnet, dass man eine Verbindung der Formel (1) einsetzt, worin mindestens einer der Substituenten $R_1$ oder $R_2$, vorzugsweise $R_1$, Chlor oder $C_1$-$C_4$-Halogenalkyl, vorzugsweise Trifluormethyl, bedeutet.

6. Verfahren nach einem der Ansprüche 1 - 5 dadurch gekennzeichnet, dass man eine Verbindung der Formel (1) einsetzt, die höchstens 2, vorzugsweise höchstens einen Substituenten aus der Gruppe $C_1$-$C_6$-Halogenalkyl, $C_1$-$C_6$-Halogenalkoxy, Nitro und Cyano enthält.

7. Verfahren nach einem der Ansprüche 1 - 6, dadurch gekennzeichnet, dass man eine Verbindung der Formel (1) einsetzt, worin die -$NHSO_2CF_3$-Gruppe in 4-Stellung steht.

8. Verfahren nach Anspruch 7, dadurch gekennzeichnet, dass man eine Verbindung der Formel (1) einsetzt, worin der Pyridinring über die 2-Stellung mit dem Sauerstoffatom verbunden ist.

9. Verfahren nach Anspruch 7 oder 8, dadurch gekennzeichnet, dass man eine Verbindung der Formel (1) einsetzt, worin eine oder beide ortho-Stellungen zur -$NHSO_2CF_3$-Gruppe durch $C_1$-$C_4$-Alkyl besetzt sind.

10. Verfahren nach Anspruch 1, dadurch gekennzeichnet, dass man eine Verbindung der Formel

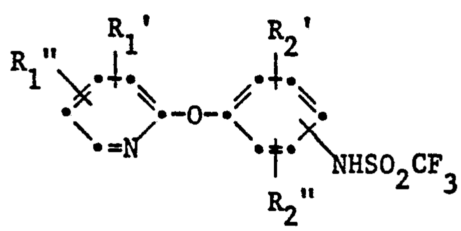

einsetzt, worin

$R_1'$ Chlor oder $C_1$-$C_4$-Halogenalkyl für Chlor oder/und Fluor steht vorzugsweise Trifluormethyl,
$R_1''$ Wasserstoff, Chlor oder $C_1$-$C_4$-Alkyl,
$R_2'$ Wasserstoff, $C_1$-$C_4$-Alkyl, $C_2$-$C_4$-Alkoxycarbonyl oder Chlor und
$R_2''$ Wasserstoff, $C_1$-$C_4$-Alkyl oder Chlor bedeuten.

11. Verfahren nach Anspruch 1, dadurch gekennzeichnet, dass man das zu schützende Material mit einer wässrigen Flotte, die eine oder mehrere in Anspruch 1 definierte Verbindungen enthält, behandelt, wobei die wässrige Flotte gegebenenfalls noch übliche Textilhilfsmittel enthalten kann.

12. Verfahren nach Anspruch 1, dadurch gekennzeichnet, dass man das zu schützende Material mit einer organischen Reinigungsflüssigkeit behandelt, die eine oder mehrere in Anspruch 1 definierte Verbindungen enthält.

13. Verfahren nach Anspruch 1, dadurch gekennzeichnet, dass man das zu schützende Material mit einem organischen Lösungsmittel besprüht, das eine oder mehrere in Anspruch 1 definierte Verbindungen enthält.

14. Verfahren nach einem der Ansprüche 1 oder 11 bis 13, dadurch gekennzeichnet, dass man auf die zu schützenden Materialien die Wirkstoffverbindungen in einer Menge von 10 bis 2'000 ppm, vorzugsweise 100 bis 1'000 ppm, bezogen auf das zu schützende Material, aufbringt.

15. Verfahren nach Anspruch 11 oder 14, dadurch gekennzeichnet, dass man Wolltextilien im Färbebad nach dem Ausziehverfahren behandelt.

16. Verfahren nach Anspruch 11 oder 14, dadurch gekennzeichnet, dass man Wolltextilien im Nachbehandlungsbad nach dem Ausziehverfahren behandelt.

17. Das nach einem der Ansprüche 1 bis 16 ausgerüstete Keratinmaterial.

18. Keratinmaterial nach Anspruch 17, vorzugsweise wollhaltige Textilien, Pelze und Felle, enthaltend 10 bis 2'000 ppm, vorzugsweise 100 bis 1'000 ppm, an Verbindungen der Formel (1).

19. Verwendung der in den Ansprüchen 1 bis 10 definierten Verbindungen zum Schützen von Keratinmaterial, insbesondere von Wolltextilien, gegen den Befall durch Keratinschädlinge.

20. Pyridyloxytrifluormethansulfonanilide der Formel

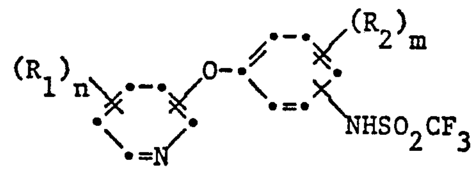

und deren Salze, worin

$R_1$ und $R_2$ unabhängig voneinander jeweils Halogen, worin Halogen Fluor, Chlor, Brom und Jod bedeutet, $C_1$-$C_6$-Halogenalkyl, $C_1$-$C_6$-Alkyl, Nitro, Cyano, $C_1$-$C_6$-Alkoxy, $C_1$-$C_6$-Halogenalkoxy oder $C_2$-$C_5$-Alkoxycarbonyl und n und m unabhängig voneinander 0 oder eine Zahl von 1 bis 3 bedeuten,

wobei, wenn n bzw. m 1, die Substituenten $R_1$ bzw. $R_2$ gleich oder verschieden sein können und wobei im Molekül mindestens ein Substituent aus der Gruppe Halogen, $C_1$-$C_6$-Halogenalkyl und $C_1$-$C_6$-Halogenalkoxy enthalten ist, und wobei für den Fall, dass $R_1$ oder $R_2$ für Halogen steht, die Summe n + m mindestens 2 beträgt.

21. Pyridyloxytriflourmethansulfonanilide nach Anspruch 20, worin $R_1$ und $R_2$ unabhängig voneinander Chlor, Flour, $C_1$-$C_4$-Halogenalkyl worin Halogen für Chlor oder/und Fluor steht $C_1$-$C_4$-Alkyl, $C_1$-$C_4$-Alkoxy Nitro oder Cyano bedeuten.

22. Pyridyloxytrifluormethansulfonanilide nach Anspruch 20 oder 21, worin die Summe n + m 1 bis 4 beträgt.

23. Pyridyloxytrifluormethansulfonanilide nach einem der Ansprüche 20 bis 22, worin mindestens einer der Substituenten $R_1$ oder $R_2$, vorzugsweise $R_1$, Chlor oder $C_1$-$C_4$-Halogenalkyl, vorzugsweise Trifluormethyl, bedeutet.

24. Pyridyloxytrifluormethansulfonailide nach einem der Ansprüche 20 - 23 die höchstens 2 vorzugsweise höchstens einen Substituenten aus der Gruppe $C_1$-$C_6$-Halogenalkyl, $C_1$-$C_6$-Halogenalkoxy, Nitro und Cyano enthalten.

25. Pyridyloxytrifluormethansulfonanilide nach einem der Ansprüche 20 bis 24, worin die $-NHSO_2CF_3$-Gruppe in 4-Stellung steht.

26. Pyridyloxytrifluormethansulfonanilide nach Anspruch 20, worin der Pyridinring über die 2-Stellung mit dem Sauerstoffatom verbunden ist.

27. Pyridyloxytrifluormethansulfonanilide nach Anspruch 25 oder 26, worin eine oder beide ortho-Stellungen zur $-NHSO_2CF_3$-Gruppe durch $C_1$-$C_4$-Alkyl besetzt sind.

28. Pyridyloxytrifluormethansulfonanilide nach Anspruch 20 der Formel

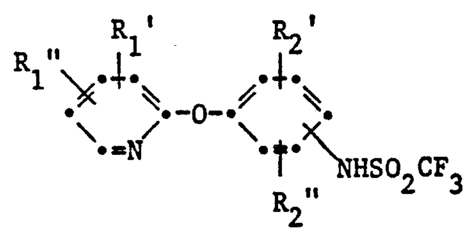

,

worin

$R_1'$ Chlor oder $C_1$-$C_4$-Halogenalkyl, worin Halogen für Chlor oder Fluor steht,
$R_1''$ Wasserstoff, Chlor oder $C_1$-$C_4$-Alkyl,
$R_2'$ Wasserstoff, $C_1$-$C_4$-Alkyl, $C_2$-$C_4$-Alkoxycarbonyl oder Chlor und
$R_2''$ Wasserstoff, $C_1$-$C_4$-Alkyl oder Chlor bedeuten,

wobei die in Anspruch 20 festgelegeten Einschränkungen bezüglich Substituentenbedeutungen gelten.

29. Verfahren zur Herstellung von Pyridyloxytrifluormethansulfonaniliden der in Anspruch 20 definierten Formel, dadurch gekennzeichnet, dass man ein Pyridyloxyanilin der Formel

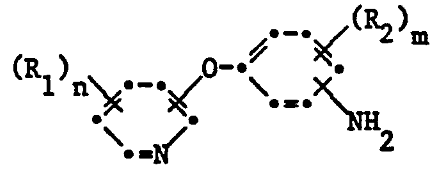

,

worin die allgemeinen Symbole die in Anspruch 20 angegebene Bedeutung haben, in Gegenwart einer Base mit Trifluormethansulfonsäureanhydrid oder einem Trifluormethansulfonsäurehalogenid umumsetzt.

30. Mittel zum Schützen bzw. Ausrüsten von Keratinmaterial bzw. keratinhaltigem Material gegen den Befall durch Keratinschädlinge, das ein oder mehrere in Anspruch 20 definierte Pyridyloxytrifluormethansulfonanilide

enthält.

31. Mittel nach Anspruch 30, das neben dem Wirkstoff noch übliche Träger und/oder Formulierungshilfsmittel enthält.

32. Mittel nach Anspruch 31, das als Träger und/oder Formulierungsmittel, organische Lösungsmittel, Wasser, Säuren, Basen, Netz-, Dispergier- und/oder Emulgiermittel enthält.

## Claims

1. A process for protecting or providing keratinous or keratin-containing material with a protective finish against attack by insects that feed on keratin, which process comprises treating the material to be protected with compounds of the formula

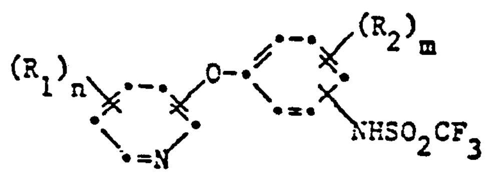

(I),

wherein

each of $R_1$ and $R_2$, independently of the other, is halogen, in which halogen is fluorine, chlorine, bromine or iodine, $C_1$-$C_6$haloalkyl, $C_1$-$C_6$alkyl, nitro, cyano, $C_1$-$C_6$alkoxy, $C_1$-$C_6$haloalkoxy or $C_2$-$C_5$alkoxycarbonyl, and

each of n and m, independently of the other, is 0 or a value from 1 to 3,

and wherein, if n or m $> 1$, the substituents $R_1$ or the substituents $R_2$ may be identical or different, and wherein at least one substituent from the group consisting of halogen, halo-$C_1$-$C_6$alkyl and halo-$C_1$-$C_6$alkoxy is present in the molecule,

or salts thereof.

2. A process according to claim 1, which comprises using a compound of formula (I), wherein each of $R_1$ and $R_2$, independently of the other, is chlorine, fluorine, $C_1$-$C_4$haloalkyl, wherein halogen is chlorine and/or fluorine, or is $C_1$-$C_4$alkyl, $C_1$-$C_4$alkoxy, $C_2$-$C_4$alkoxy-carbonyl, nitro or cyano.

3. A process according to claim 1, which comprises using a compound of formula (I), wherein the sum of n + m is at least 2 if $R_1$ or $R_2$ is halogen.

4. A process according to any one of claims 1 to 3, which comprises using a compound of formula (I), wherein the sum of n + m is 1 to 4.

5. A process according to any one of claims 1 to 4, which comprises using a compound of formula (I), wherein at least one of the substituents $R_1$ and $R_2$, preferably $R_1$, is chlorine or $C_1$-$C_4$haloalkyl, preferably trifluoromethyl.

6. A process according to any one of claims 1 to 5, which comprises using a compound of formula (I) which contains not more than 2, preferably not more than one, substituent(s) from the group consisting of $C_1$-$C_6$haloalkyl, $C_1$-$C_6$haloalkoxy, nitro and cyano.

7. A process according to any one of claims 1 to 6, which comprises using a compound of formula (I), wherein the $-NHSO_2CF_3$ group is in the 4-position.

8. A process according to claim 7, which comprises using a compound of formula (I), wherein the pyridine ring is attached in the 2-position to the oxygen atom.

9. A process according to claim 7 or claim 8, which comprises using a compound of formula (I) which carries $C_1$-$C_4$alkyl in one or both ortho-positions relative to the $-NHSO_2CF_3$ group.

10. A process according to claim 1, which process comprises using a compound of the formula

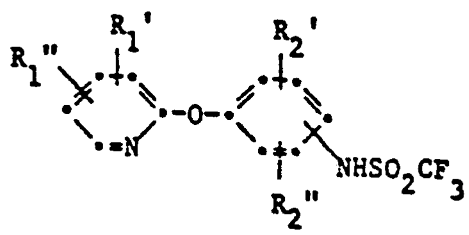

wherein

$R_1'$ is chlorine or $C_1$-$C_4$haloalkyl, wherein halogen is chlorine and/or fluorine, preferably trifluoromethyl,

$R_1''$ is hydrogen, chlorine or $C_1$-$C_4$alkyl,

$R_2'$ is hydrogen, $C_1$-$C_4$alkyl, $C_2$-$C_4$alkoxycarbonyl or chlorine and

$R_2''$ is hydrogen, $C_1$-$C_4$alkyl or chlorine.

11. A process according to claim 1, which process comprises treating the material to be protected with an

aqueous liquor containing one or more of the compounds defined in claim 1, it being possible for the aqueous liquor optionally also to contain conventional textile assistants.

12. A process according to claim 1, which comprises treating the material to be protected with an organic cleaning liquid containing one or more of the compounds defined in claim 1.

13. A process according to claim 1, which comprises spraying the material to be protected with an organic solvent containing one or more of the compounds defined in claim 1.

14. A process according to any one of claims 1 or 11 to 13, which comprises applying to the materials to be protected the active compounds in an amount of 10 to 2000 ppm, preferably 100 to 1000 ppm, based on the material to be protected.

15. A process according to claim 11 or claim 14, which comprises treating woollen textiles in a dye bath by the exhaust process.

16. A process according to claim 11 or claim 14, which comprises treating woollen textiles in the aftertreatment bath by the exhaust process.

17. Keratinous material finished according to any one of claims 1 to 16.

18. Keratinous material according to claim 17, preferably textiles containing wool, furs and pelts, containing 10 to 2000 ppm, preferably 100 to 1000 ppm, of a compound of formula (I).

19. Use of the compounds defined in claims 1 to 10 for protecting keratinous material, especially woollen textiles, against attack by pests that damage keratin.

20. Pyridyloxytrifluoromethanesulfonanilides of the formula

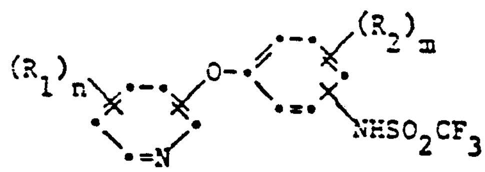

wherein

each of $R_1$ and $R_2$, independently of the other, is halogen, in which halogen is fluorine, chlorine, bromine or iodine, $C_1$-$C_6$haloalkyl, $C_1$-$C_6$alkyl, nitro, cyano, $C_1$-$C_6$alkoxy, $C_1$-$C_6$haloalkoxy or $C_2$-$C_5$alkoxycarbonyl, and

each of n and m, independently of the other, is 0 or a value from 1 to 3,

wherein, if n or m $>$ 1, the substituents $R_1$ or the substituents $R_2$ may be identical or different, and wherein at least one substituent from the group consisting of halogen, $C_1$-$C_6$haloalkyl and $C_1$-$C_6$haloalkoxy is present in the molecule, and the sum of n + m is at least 2 if $R_1$ or $R_2$ is halogen, and the salts thereof.

21. Pyridyloxytrifluoromethanesulfonanilides according to claim 20, wherein each of $R_1$ and $R_2$, independently of the other, is chlorine, fluorine, $C_1$-$C_4$haloalkyl, wherein halogen is chlorine and/or fluorine, or is $C_1$-$C_4$alkyl, $C_1$-$C_4$alkoxy, nitro or cyano.

22. Pyridyloxytrifluoromethanesulfonanilides according to claim 20 or claim 21, wherein the sum of n + m is 1 to 4.

23. Pyridyloxytrifluoromethanesulfonanilides according to any one of claims 20 to 22, wherein at least one of the substituents $R_1$ and $R_2$, preferably $R_1$, is chlorine or $C_1$-$C_4$haloalkyl, preferably trifluoromethyl.

24. Pyridyloxytrifluoromethanesulfonanilides according to any one of claims 20 to 23, which contain not more than 2, preferably not more than one, substituent(s) from the group consisting of $C_1$-$C_6$haloalkyl, $C_1$-$C_6$haloalkoxy, nitro and cyano.

25. Pyridyloxytrifluoromethanesulfonanilides according to any one of claims 20 to 24, wherein the $-NHSO_2CF_3$ group is in the 4-position.

26. Pyridyloxytrifluoromethanesulfonanilides according to claim 20, wherein the pyridine ring is attached in the 2-position to the oxygen atom.

27. Pyridyloxytrifluoromethanesulfonanilides according to claim 25 or claim 26, which carries $C_1$-$C_4$alkyl in one or both ortho-positions relative to the $-NHSO_2CF_3$ group.

28. Pyridyloxytrifluoromethanesulfonanilides according to claim 20 of the formula

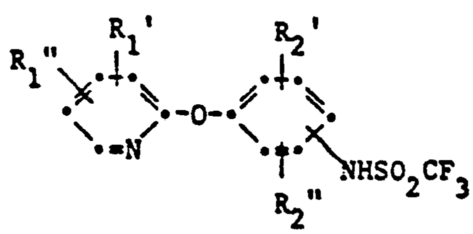

wherein

$R_1'$ is chlorine or $C_1$-$C_4$haloalkyl, wherein halogen is chlorine or fluorine,

$R_1''$ is hydrogen, chlorine or $C_1$-$C_4$alkyl,

$R_2'$ is hydrogen, $C_1$-$C_4$alkyl, $C_2$-$C_4$alkoxycarbonyl or chlorine and

$R_2''$ is hydrogen, $C_1$-$C_4$alkyl or chlorine, and with the restrictions stated in claim 20 with regard to the meanings of the substituents.

29. A process for the preparation of pyridyloxytrifluoromethanesulfonanilides of the formula as defined in claim 20, which process comprises reacting a pyridyloxyaniline of the formula

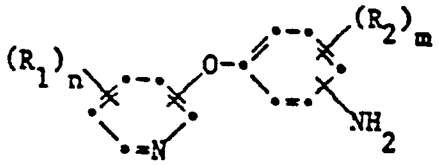

wherein the general symbols have the meanings given in claim 20, with trifluoromethanesulfonic anhydride or with a trifluoromethanesulfonyl halide in the presence of a base.

30. A composition for protecting or providing keratinous or keratin-containing material with a protective finish against attack by pests that damage keratin, said composition containing one or more of the pyridyloxytrifluoromethanesulfonanilides as defined in claim 20.

31. A composition according to claim 30, which, in addition to the active ingredient, contains conventional carriers and/or formulation assistants.

32. A composition according to claim 31, which contains organic solvents, water, acids, bases, wetting, dispersing and/or emulsifying agents as carriers and/or formulation agents.

## Revendications

1. Procédé pour protéger ou apprêter des matières consistant en totalité ou en partie en kératine contre l'attaque par les insectes dévorant la kératine, caractérisé en ce que l'on traite la matière à protéger par des composés de formule

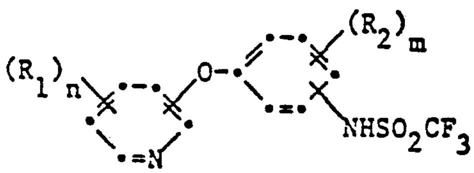

(1)

ou leurs sels dans lesquels

$R_1$ et $R_2$ représentent chacun, indépendamment l'un de l'autre, un halogène, cet halogène consistant en fluor, chlore, brome ou iode, un groupe halogénoalkyle en C 1-C 6, alkyle en C 1-C 6, nitro, cyano, alcoxy en C 1-C 6, halogénoalcoxy en C 1-C 6 ou alcoxycarbonyle en C 2-C 5 et

n et m sont égaux chacun, indépendamment l'un de l'autre, à 0 ou à un nombre allant de 1 à 3,

étant spécifié que lorsque n ou m est supérieur à 1, les substituants $R_1$ et $R_2$ peuvent être identiques ou différents, et que la molécule contient au moins un substituant pris dans le groupe formé par les halogènes, les groupes halogénoalkyle en C 1-C 6 et halogénoalcoxy en C 1-C 6.

2. Procédé selon la revendication 1, caractérisé en ce que l'on utilise un composé de formule (1) dans laquelle $R_1$ et $R_2$ représentent chacun, indépendamment l'un de l'autre, le chlore, le fluor, un groupe halogénoalkyle en C 1-C 4 dans lequel l'halogène consiste en chlore ou fluor, un groupe alkyle en C 1-C 4, alcoxy en C 1-C 4, alcoxycarbonyle en C 2-C 4, nitro ou cyano.

3. Procédé selon la revendication 1, caractérisé en ce que l'on utilise un composé de formule (1) dans lequel lorsque $R_1$ ou $R_2$ représente un halogène, la somme n + m est égale au moins à 2.

4. Procédé selon l'une des revendications 1 à 3, caractérisé en ce que l'on utilise un composé de formule (1) dans laquelle la somme n + m a une valeur de 1 à 4.

5. Procédé selon l'une des revendications 1 à 4, caractérisé an ce que l'on utilise un composé de formule (1) dans laquelle l'un au moins des substituants $R_1$ ou $R_2$, de préférence $R_1$, consiste en chlore ou en un groupe halogénoalkyle en C 1-C 4, de préférence trifluorométhyle.

6. Procédé selon l'une des revendications 1 à 5, caractérisé en ce que l'on utilise un composé de formule (1) contenant au maximum 2, de préférence au moins 1 substituant de la classe des groupes halogénoalkyle en C 1-C 6, halogénoalcoxy en C 1-C 6, nitro et cyano.

7. Procédé selon l'une des revendications 1 à 6, caractérisé en ce que l'on utilise un composé de formule (1) dans laquelle le groupe $-NHSO_2CF_3$ est en position 4.

8. Procédé selon la revendication 7, caractérisé en ce que l'on utilise un composé de formule (1) dans laquelle le cycle pyridine est relié avec l'atome d'oxygène par la position 2.

9. Procédé selon la revendication 7 ou 8, caractérisé en ce que l'on utilise un composé de formule (1) dans laquelle une position ortho ou les deux positions ortho du groupe $-NHSO_2CF_3$ sont occupées par les groupes

alkyle en C 1-C 4.

10. Procédé selon la revendication 1, caractérisé en ce que l'on utilise un composé de formule

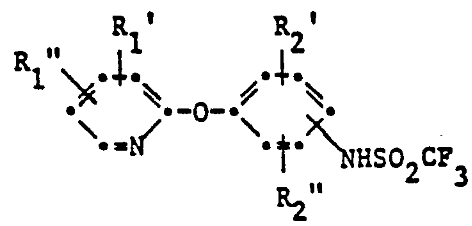

dans laquelle

$R'_1$ représente le chlore ou un groupe halogénoalkyle en C 1-C 4 dans lequel l'halogéne est le chlore et/ou le fluor, de préférence un groupe trifluorométhyle,

$R''_1$ représente l'hydrogène, le chlore ou un groupe alkyle en C 1-C 4,

$R'_2$ représente l'hydrogène, un groupe alkyle en C 1-C 4, alcoxycarbonyle en C 2-C 4 ou le chlore, et

$R''_2$ représente l'hydrogène, un groupe alkyle en C 1-C 4 ou le chlore.

11. Procédé selon la revendication 1, caractérisé en ce que l'on traite la matière à protéger par un bain aqueux contenant un ou plusieurs composés définis dans la revendication 1, le bain aqueux contenant encore le cas échéant des produits auxiliaires textiles usuels.

12. Procédé selon la revendication 1, caractérisé en ce que l'on traite la matière à protéger par un liquide organique de nettoyage contenant un ou plusieurs composés définis dans la revendication 1.

13. Procédé selon la revendication 1, caractérisé en ce que l'on pulvérise la matière à protéger par un solvant organique contenant un ou plusieurs composés définis dans la revendication 1.

14. Procédé selon l'une des revendications 1 ou 11 à 13, caractérisé en ce que l'on applique les substances actives sur les matières à protéger en quantités de 10 à 2000 ppm, de préférence de 100 à 1000 ppm, par rapport à la matière à protéger.

15. Procédé selon la revendication 11 ou 14, caractérisé en ce que l'on traite des matières textiles en laine dans le bain de teinture par le procédé par épuisement.

16. Procédé selon la revendication 11 ou 14, caractérisé en ce que l'on traite les matières textiles de laine dans le bain de traitement subséquent par la technique par épuisement.

17. La matière kératinique apprêtée selon l'une des revendications 1 à 16.

18. Matière kératinique selon la revendication 17, de préférence matière textile contenant de la laine, pelleterie ou fourrure, contenant 10 à 2000 ppm, de préférence 100 à 1000 ppm, de composés de formule (1).

19. Utilisation des composés définis dans les revendications 1 à 10, pour la protection des matières kératiniques, en particulier des textiles en laine, contre l'attaque par les parasites de la kératine.

20. Pyridyloxytrifluorométhane-sulfonanilides de formule

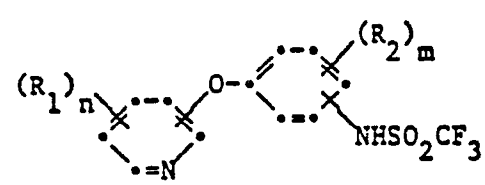

et leurs sels, dans lesquels

$R_1$ et $R_2$ représentent chacun, indépendamment l'un de l'autre, un halogéne, cet halogène consistant lui-même en fluor, chlore, brome ou iode, un groupe halogénoalkyle en C 1-C 6, alkyle en C 1-C 6, nitro, cyano, alcoxy en C 1-C 6, halogénoalcoxy en C 1-C 6 ou alcoxycarbonyle en C 2-C 5 et

n et m représentent chacun, indépendamment l'un de l'autre, 0 ou un nombre allant de 1 à 3,

étant spécifié que lorsque n ou m est supérieur à 1, les substituants $R_1$ et $R_2$ peuvent être identiques ou différents, que la molécule contient au moins un substituant de la classe formée par les halogènes, les groupes halogénoalkyle en C 1-C 6 et halogénoalcoxy en C 1-C 6 et que, dans le cas où $R_1$ ou $R_2$ représente un halogène, la somme n + m est égale au moins à 2.

21. Pyridyloxytrifluorométhane-sulfonanilides selon la revendication 20, dans lesquels $R_1$ et $R_2$ représentent chacun, indépendamment l'un de l'autre, le chlore, le fluor, un groupe halogénoalkyle en C 1-C 4 dans lequel l'halogéne est le chlore et/ou le fluor, un groupe alkyle en C 1-C 4, alcoxy en C 1-C 4, nitro ou cyano.

22. Pyridyloxytrifluorométhane-sulfonanilides selon la revendication 20 ou 21, dans lesquels la somme n + m a une valeur de 1 à 4.

23. Pyridyloxytrifluorométhane-sulfonanilides selon l'une des revendications 20 à 22, dans lesquels l'un au moins des substituants $R_1$ ou $R_2$, de préférence $R_1$, représente le chlore ou un groupe halogénoalkyle en C 1-C 4, de préférence un groupe trifluorométhyle.

24. Pyridyloxytrifluorométhane-sulfonanilides selon l'une des revendications 20 à 23, contenant au maximum deux, de préférence au maximum un substituant de la classe des groupes halogénoalkyle en C 1-C 6,

halogénoalcoxy en C 1-C 6, nitro et cyano.

25. Pyridyloxytrifluorométhane-sulfonanilides selon l'une des revendications 20 à 24, dans lesquels le groupe $-NHSO_2CF_3$ est en position 4.

26. Pyridyloxytrifluorométhane-sulfonanilides selon la revendication 20, dans lesquels le cycle pyridine est relié à l'atome d'oxygène par la position 2.

27. Pyridyloxytrifluorométhane-sulfonanilides selon la revendication 25 ou 26, dans lesquels une position ortho ou les deux positions ortho du groupe $-NHSO_2CF_3$ sont occupées par des groupes alkyle en C 1-C 4.

28. Pyridyloxytrifluorométhane-sulfonanilides selon la revendication 20, de formule

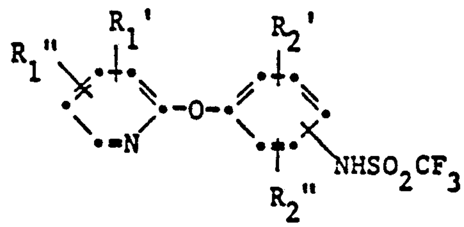

,

dans laquelle

R'$_1$ représente le chlore ou un groupe halogénoalkyle en C 1-C 4 dans lequel l'halogène est le chlore ou le fluor,

R"$_1$ représente l'hydrogène, le chlore ou un groupe alkyle en C 1-C 4,

R'$_2$ représente l'hydrogène, un groupe alkyle en C 1-C 4, alcoxycarbonyle en C 2-C 4 ou le chlore, et

R"$_2$ représente l'hydrogène, un groupe alkyle en C 1-C 4 ou le chlore,

les limitations indiquées dans la revendication 20 concernant les significations des substituants restant valables.

29. Procédé de préparation des pyridyloxytrifluorométhane-sulfonanilides de formule définie dans la revendication 20, caractérisé en ce que l'on fait réagir une pyridyloxyaniline de formule

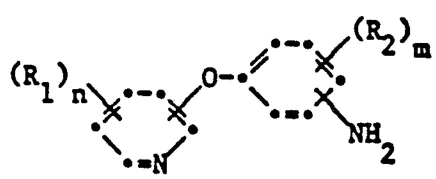

,

dans laquelle les symboles généraux ont les significations indiquées dans la revendication 20, en présence d'une base, avec l'anhydride trifluorométhane-sulfonique ou un halogénure d'acide trifluorométhanesulfonique.

30. Produit pour protéger ou apprêter les matières consistant en totalité ou en partie en kératine contre l'attaque par les parasites de la kératine, contenant un ou plusieurs pyridyloxytrifluorométhanesulfonanilides définis dans la revendication 20.

31. Produit selon la revendication 30, contenant, avec la substance active, des véhicules et/ou produits auxiliaires de formulation usuels.

32. Produit selon la revendication 31, contenant en tant que véhicules et/ou produits auxiliaires de formulation, des solvants organiques, de l'eau, des acides, des bases, des agents mouillants, dispersants et/ou émulsionnants.